# EUROPEAN PATENT APPLICATION

(11) **EP 3 290 084 A1**
(43) Date of publication of application: **07.03.2018**
(21) Application number: 17184020.0
(22) Date of filing: 31.07.2017
(51) Int. Cl.: A61N 1/36, A61N 1/04

(54) **CONTROL DEVICE FOR ELECTRICAL STIMULATION APPARATUS, ELECTRICAL STIMULATION APPARATUS, AND PEDALING EXERCISE SYSTEM**

(30) Priority: 30.08.2016 JP 2016168517
(71) Applicant: Panasonic Intellectual Property Management Co., Ltd., Osaka-shi, Osaka 540-6207 (JP)
(72) Inventor: NISHIMURA, Yoshinari, Osaka-shi, Osaka 540-6207 (JP); SHINOMIYA, Yoichi, Osaka-shi, Osaka 540-6207 (JP)
(74) Representative: Grünecker Patent- und Rechtsanwälte PartG mbB

(57) **Abstract**

A control device for an electrical stimulation apparatus in the present disclosure includes a control section that adjusts an output of an electrode. The electrode gives electrical stimulation to a predetermined part of a body doing a pedaling exercise. The predetermined part is at least one of a leg and an arm. The control section adjusts the output of the electrode in such a way that the electrical stimulation is given to the predetermined part in relation to an exercise unit of the pedaling exercise. Furthermore, the control section adjusts strength of the electrical stimulation related to the exercise unit, in accordance with an amount of a physical activity of the body during the pedaling exercise. In this way, electrical stimulation can be given to a predetermined part in a variable manner during the pedaling exercise.

## Description

### BACKGROUND

### 1. Technical Field

The present disclosure relates to a control device for an electrical stimulation apparatus that electrically stimulates predetermined parts of a user's body while a user is doing pedaling exercise, to an electrical stimulation apparatus, and to a pedaling exercise system.

### 2. Description of the Related Art

Unexamined Japanese Patent Publication No. 2003-144556 discloses an electrical stimulation apparatus that helps a user do pedaling exercise or intensifies the pedaling exercise. More specifically, this electrical stimulation apparatus electrically stimulates the lower limbs, based on locations or rotation angles of the pedals.

### SUMMARY

In a state in which a user does pedaling exercise, even if a number of revolutions of pedals increases, an application pattern of a voltage to be applied to respective electrodes is constant. However, for example, different patterns of voltage are preferably applied to the electrodes right and long after the user starts pedaling. In this case, one example of the voltage pattern may be an increasing rate at which the voltage rises to its maximum.

A control device for an electrical stimulation apparatus according to one aspect of the present disclosure includes a control section that adjusts an output of an electrode. The electrode gives electrical stimulation to a predetermined part of a body doing a pedaling exercise. The predetermined part is at least one of a leg and an arm. The control section adjusts the output of the electrode in such a way that the electrical stimulation is given to the predetermined part in relation to an exercise unit of the pedaling exercise. In addition, the control section adjusts strength of the electrical stimulation related to the exercise unit, in accordance with an amount of a physical activity of the body during the pedaling exercise.

The above control device for an electrical stimulation apparatus, an electrical stimulation apparatus, and a pedaling exercise system can electrically stimulate the predetermined part in a variable manner during the pedaling exercise.

### BRIEF DESCRIPTION OF THE DRAWINGS

FIG. 1 is a perspective view of a pedaling exercise system according to an exemplary embodiment;
FIG. 2 is a block diagram of the electrical stimulation apparatus in the pedaling exercise system illustrated in FIG. 1;
FIG. 3 is a graph illustrating changes in an angle of a knee joint and an angular velocity acquired after a filtering process;
FIG. 4 is a graph illustrating a frequency response in the filtering process to be performed by the control section;
FIG. 5 is a graph illustrating a phase characteristic in the filtering process to be performed by the control section;
FIG. 6 is a graph illustrating one example of an angular velocity acquired after the filtering process when the crank rotates at a low speed;
FIG. 7 is a graph illustrating one example of an angular velocity acquired after the filtering process when the crank rotates at a high speed;
FIG. 8 is a map for calculation of a timing of a voltage application to a first electrode;
FIG. 9 is a map for calculation of a timing of a voltage application to a second electrode;
FIG. 10 is a graph illustrating a relationship between a number of revolutions of the crank and an applied voltage;
FIG. 11 is a graph illustrating the voltages applied to the respective electrodes;
FIG. 12 is a map illustrating the relationship between a rotation speed of the crank and an increasing rate of the applied voltage;
FIG. 13 is a flowchart of a first process to be performed by the control section;
FIG. 14 is a flowchart of a second process to be performed by the control section; and
FIG. 15 is a flowchart of a third process to be performed by the control section.

### DETAILED DESCRIPTION

### (One Example of Aspects of Control Device for Electrical Stimulation Apparatus, Electrical Stimulation Apparatus, and Pedaling Exercise System)

A control device for an electrical stimulation apparatus according to an aspect of the present disclosure includes a control section that adjusts an output of an electrode. The electrode gives electrical stimulation to a predetermined part of a body doing a pedaling exercise. The predetermined part is at least one of a leg and an arm. The control section adjusts the output of the electrode so that the electrical stimulation is given to the predetermined part in relation to an exercise unit of the pedaling exercise. In addition, the control section adjusts strength of the electrical stimulation related to the exercise unit, in accordance with an amount of a physical activity of the body during the pedaling exercise.

The above configuration varies an output mode of the electrodes in relation to the exercise unit during the pedaling exercise. This can electrically stimulate the predetermined part in a variable manner during the pedaling exercise. The amount of the physical activity may be estimated based on the number of revolutions of the predetermined part during the pedaling exercise or a time of the pedaling exercise.

According to one example of the control device for an electrical stimulation apparatus, the control section adjusts the strength of the electrical stimulation related to the exercise unit so that the strength of the electrical stimulation increases according as the amount of the physical activity of the body increases during the pedaling exercise.

As a result, when electrical stimulation is output to the predetermined part, the electrical stimulation gradually increases, and thus, user discomfort caused by electrical stimulation can be made to be less than a case where strong electrical stimulation is given to the predetermined part when the output of the electrical stimulation is started.

According to one example of the control device for an electrical stimulation apparatus, when the strength of the electrical stimulation is set by an operation section, the control section adjusts the strength of the electrical stimulation related to the exercise unit so that an initial value of the electrical stimulation related to the exercise unit becomes lower than the set strength and that the strength of the electrical stimulation gradually increases toward the set strength according as the amount of the physical activity of the body increases.

According to one example of the control device for an electrical stimulation apparatus, the control section adjusts an increasing rate of the strength of the electrical stimulation when the strength of the electrical stimulation is gradually increased toward the set strength, in accordance with a rotation speed of a crank in a pedaling exercise machine.

The above configuration can suppress the risk that a period in which the electrical stimulation is being output at the set strength varies depending on the rotation speed of the crank, during the pedaling exercise.

According to one example of the control device for an electrical stimulation apparatus, the control section causes the electrode to output the electrical stimulation related to the exercise unit by applying a pulse voltage to the electrode.

This configuration varies a mode of applying the pulse voltage to the electrode. Consequently, electrical stimulation to be given to the predetermined part can be finely adjusted during the pedaling exercise.

According to one example of the control device for an electrical stimulation apparatus, the control section gradually increases the pulse voltage during a first half of a period of the exercise unit, the first half of the period containing a starting point of the exercise unit.

This configuration increases the electrical stimulation to be output to the predetermined part every time when a pulse voltage is applied to the electrode. User discomfort caused by electrical stimulation can be thus made to be less than the case where strong electrical stimulation is given to the predetermined part when the output of the electrical stimulation is started.

According to one example of the control device for an electrical stimulation apparatus, the control section adjusts the increasing rate of the voltage when the pulse voltage is gradually increased in accordance with a rotation speed of the crank of the pedaling exercise machine.

The above configuration can reduce a risk that the period in which the electrical stimulation is output at the set strength varies depending on the rotation speed of the crank, during the application of the pulse voltage.

The above electrical stimulation apparatus according to one aspect includes: the above control device for an electrical stimulation apparatus; and the electrode.

The above pedaling exercise system according to one aspect includes the above electrical stimulation apparatus, and the pedaling exercise machine.

### EXEMPLARY EMBODIMENT

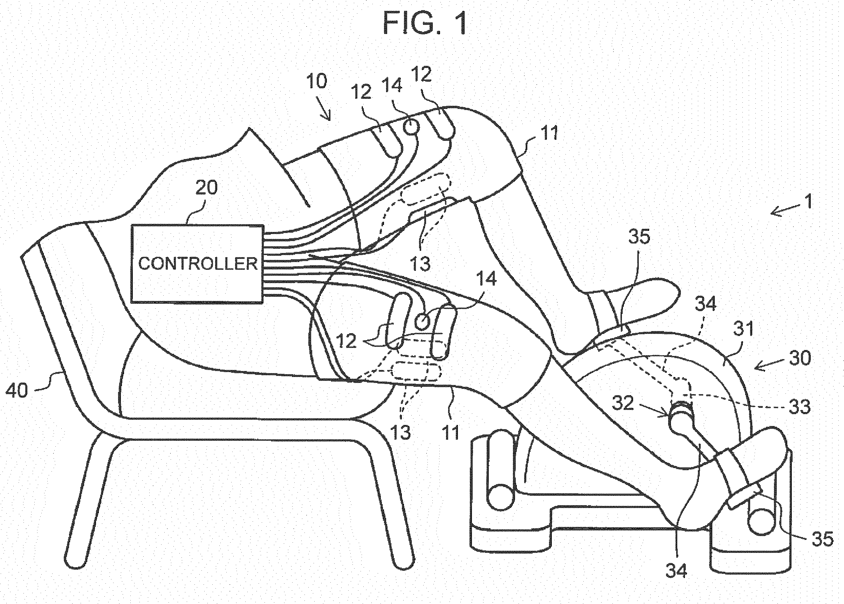

As illustrated in FIG. 1, pedaling exercise system 1 includes electrical stimulation apparatus 10, pedaling exercise machine 30, and a seat 40. Stimulation apparatus 10 is attached to user's lower limbs. The user does pedaling exercise using pedaling exercise machine 30 while sitting on seat 40. The configuration of pedaling exercise system 1 may be modified appropriately. As a first example, pedaling exercise machine 30 is integrated with seat 40. As a second example, the pedaling exercise system 1 further includes a handle to be grabbed by the user's hand during the pedaling exercise. As a third example, seat 40 is provided with a mechanism that adjusts a height of seat 40. With this mechanism, the height of seat 40 can be adjusted so that the user does the pedaling exercise easily.

Electrical stimulation apparatus 10 electrically stimulates his/her lower limbs in accordance with the user's pedaling exercise. Electrical stimulation apparatus 10 includes supporters 11 and controller 20. Supporters 11 are attached to right and left lower limbs. Controller 20 is one example of a control device that controls electrical stimulation apparatus 10.

Each supporter 11 is provided with two first electrodes 12, two second electrodes 13, and angular velocity detector 14. First electrodes 12 and second electrodes 13 are disposed with a space being left therebetween. Two first electrodes 12 are disposed with a space being left therebetween. Two second electrodes 13 are disposed with a space being left therebetween. Angular velocity detector 14 is disposed closer to first electrodes 12 than second electrodes 13. More specifically, angular velocity detector 14 is disposed between two first electrodes 12. In this case, each supporter 11 may have any number of first electrodes 12 and second electrodes 13. As one example, each supporter 11 may have one or not less than three first electrodes 12 and/or second electrodes 13.

When supporters 11 are attached to the lower limbs, first electrodes 12 are disposed at locations related to the quadriceps femorises of the lower limbs and second electrodes 13 are disposed at locations related to the biceps femorises of the lower limbs, for example. Angular velocity detectors 14 are disposed at locations related to the quadriceps femorises, for example. During the pedaling exercise, first electrodes 12 electrically stimulate the quadriceps femorises, second electrodes 13 electrically stimulate the biceps femorises, and angular velocity detectors 14 detect angular velocities of the quadriceps femorises. Angular velocity detectors 14 output detection signals according to the detected angular velocities to controller 20.

Controller 20 is electrically connected to supporters 11 by wires so as to be electrically connected to first electrodes 12, second electrodes 13, and angular velocity detectors 14. Controller 20 controls a mode of applying voltages to first electrodes 12 and second electrodes 13, based on detection signals from angular velocity detectors 14. Note that controller 20 may be wirelessly electrically connected to first electrodes 12, second electrodes 13, and angular velocity detectors 14.

Pedaling exercise machine 30 includes main body 31 and crank 32, for example. The main body 31 is installed on a floor, and crank 32 is provided with pedals 35. Main body 31 is provided with an electric motor (not illustrated) that rotates crank 32. Crank 32 includes crank shaft 33 and a pair of crank arms 34. Crank shaft 33 penetrates main body 31 with axial both ends protruding from main body 31. Crank arms 34 are connected to both ends of crank shaft 33 and extend perpendicularly to an axial direction of crank shaft 33. Pedals 35 are mounted to ends of crank arms 34.

Pedaling exercise machine 30 is provided with two operation modes including an automatic operation mode and a passive operation mode. In the automatic operation mode, crank 32 is rotated by the electric motor. In the passive operation mode, crank 32 is rotated by the pedaling exercise done by a user. During the passive operation mode, the electric motor stops, for example. Pedaling exercise machine 30 does not necessarily have to operate in the automatic operation mode. In this case, the electric motor is removed from pedaling exercise machine 30.

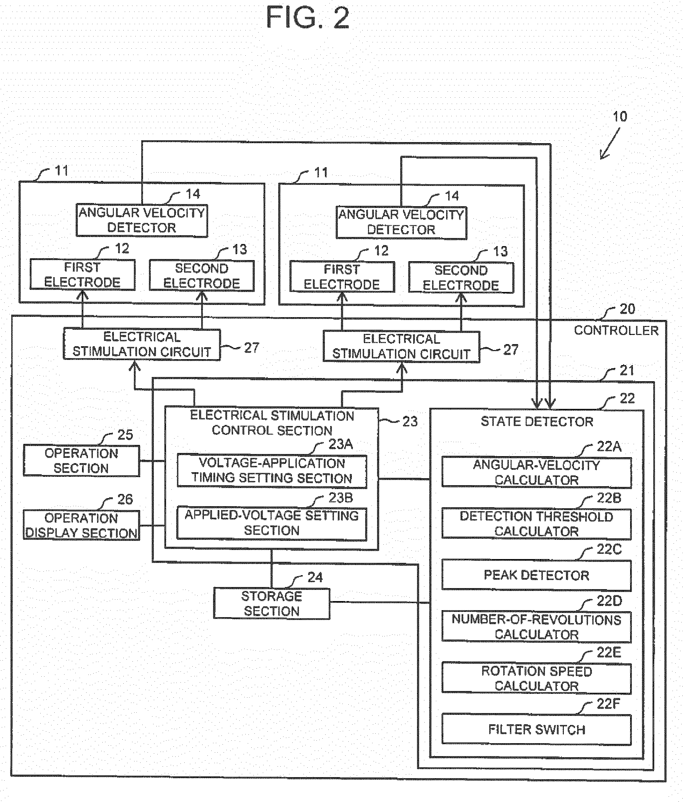

With reference to FIG. 2, an electrical configuration of electrical stimulation apparatus 10 will be described.

Controller 20 includes control section 21, storage section 24, operation section 25, operation display section 26, and electrical stimulation circuits 27.

Control section 21 executes predetermined control programs. Control section 21 includes a central processing unit (a CPU) or a micro processing unit (an MPU). Control section 21 outputs control signals to electrical stimulation circuits 27 in order to control electrical stimulation circuits 27. Accordingly, control section 21 adjusts outputs from first electrodes 12 and second electrodes 13, which electrically stimulate lower limbs (legs) doing the pedaling exercise.

Storage section 24 stores information to be used for various control programs and control processes. Storage section 24 includes a random access memory (a RAM) and a read only memory (a ROM), for example. Storage section 24 may be incorporated into control section 21.

Operation section 25 allows a user to set voltages to be applied to first electrodes 12 and second electrodes 13 via electrical stimulation circuits 27, a period of the pedaling exercise during which the electrical stimulation is given, and an upper limit of a number of times that the electrical stimulation is given. Operation section 25 enables setting of voltages to be applied to first electrodes 12 and second electrodes 13 independently from each other. Hereinafter, the term "set voltage" refers to a voltage being strength of electrical stimulation set through operation section 25 among voltages applied to first and second electrodes 12, 13.

Operation display section 26 includes a liquid crystal panel, for example, and displays the contents set through operation section 25 and other information.

Electrical stimulation circuits 27 control the application of voltages to first electrodes 12 and second electrodes 13 in supporters 11, based on control signals from control section 21. Each electrical stimulation circuit 27 applies pulse voltages to first and second electrodes 12, 13. As a result, electric currents are output from first electrodes 12 and second electrodes 13.

Control section 21 includes state detector 22 and electrical stimulation control section 23. State detector 22 detects states of lower limbs, and a number of revolutions and a rotation speed of crank 32. Electrical stimulation control section 23 controls timings at which the voltages are applied to electrodes 12, 13 in supporters 11 and levels of the applied voltages.

State detector 22 includes angular-velocity calculator 22A, detection threshold calculator 22B, peak detector 22C, number-of-revolutions calculator 22D, rotation speed calculator 22E, and filter switch 22F. State detector 22 is electrically connected to electrical stimulation control section 23 and storage section 24.

Angular-velocity calculator 22A executes a filtering process on detection signals from each angular velocity detector 14 using a filter set by filter switch 22F so as to remove noise from the detection signals to smooth the detection signals. Angular-velocity calculator 22A calculates an angular velocity (referred below as "angular velocities AC") smoothed in such a manner. One example of this filtering process is a moving-average filtering process.

The detection threshold calculator 22B calculates a detection threshold which is used to detect a peak of angular velocity AC, based on angular velocity AC. For example, detection threshold calculator 22B acquires a peak of angular velocity AC for each rotation of crank 32 (see FIG. 1) within a first period. The first period is a period over which the number of revolutions of crank 32 is equal to or less than a predetermined number of revolutions RX. The peak of angular velocity AC is a maximum value of angular velocity AC within a range of transition from an increasing state to a decreasing state. Detection threshold calculator 22B then sets peak threshold XP, which is used to detect the peak of angular velocity AC, based on a plurality of peaks of angular velocity AC. In one example, detection threshold calculator 22B sets the average of the plurality of peak values to peak threshold XP.

Peak detector 22C compares angular velocity AC and peak threshold XP. Then, based on the comparison result, peak detector 22C detects a peak of angular velocity AC within a second period being a period that comes after the number of revolutions of crank 32 exceeds the predetermined number of revolutions RX.

Number-of-revolutions calculator 22D calculates the number of revolutions of crank 32, based on the number of peaks of angular velocity AC. Number-of-revolutions calculator 22D resets the number of revolutions of crank 32 which has been accumulated, when a rotation speed of crank 32 becomes zero.

Rotation speed calculator 22E calculates a rotation speed of crank 32, based on angular velocity AC. A correlation holds between an angular velocity of a lower limb and a rotation speed of crank 32. Therefore, storage section 24 prestores information regarding the relationship between one cycle of angular velocity AC and a rotation speed of the crank 32, for example in a function or map format. Based on the information regarding the relationship between one cycle of angular velocity AC and a rotation speed of the crank 32, rotation speed calculator 22E calculates a rotation speed of crank 32 from the angular velocity calculated by angular-velocity calculator 22A. Note that, for example, rotation speed calculator 22E calculates one cycle of angular velocity AC, based on neighboring peaks of angular velocity AC.

Filter switch 22F includes a first filter and a second filter. A frequency response curve is steeper in the first filter than the second filter. The first filter provides a larger amount of reference data than the second filter. Each of the first and second filters is a low-pass filter. Note that each of the first and second filters may be a high-pass filter, a band-pass filter, or a resistor-capacitor (RC) filter.

Filter switch 22F switches between the first and second filters, based on the number of revolutions of crank 32. When the number of revolutions of crank 32 falls within a first range, filter switch 22F switches over to the first filter. When the number of revolutions of crank 32 falls within a second range that is wider than the first range, filter switch 22F switches over to the second filter. As one example, when the number of revolutions of crank 32 is equal to or less than the predetermined number of revolutions RX, filter switch 22F switches over to the first filter. When the number of revolutions of crank 32 exceeds the predetermined number of revolutions RX, filter switch 22F switches over to the second filter.

Immediately after a user starts doing the pedaling exercise, an exercise pace tends to be so unstable that noise is easily caused in a detection signal from each angular velocity detector 14. Therefore, when the number of revolutions of crank 32 is equal to or less than the number of revolutions RX, angular-velocity calculator 22A performs the filtering process using the first filter, thereby removing noise from the detection signal of angular velocity AC. Therefore, angular velocity AC is accurately calculated. After a considerable time has passed since a user has started doing the pedaling exercise, an exercise pace tends to be so stable that noise is hardly caused in a detection signal from each angular velocity detector 14. Therefore, when the number of revolutions of crank 32 exceeds the predetermined number of revolutions RX, angular-velocity calculator 22A performs the filtering process using the second filter. Performing this filtering process involves placing a lighter load on control section 21 than performing the filtering process using the first filter.

Electrical stimulation control section 23 includes voltage-application timing setting section 23A and applied-voltage setting section 23B.

Voltage-application timing setting section 23A adjusts timings at which electrodes 12, 13 give electrical stimulation, in accordance with angular velocity AC. Adjusting the timings in this manner enables legs, which are predetermined parts of a body of a user doing the pedaling exercise, to be electrically stimulated when the legs are in a predetermined motion state. In one example, voltage-application timing setting section 23A applies a voltage to one of first and second electrodes 12, 13 which is related to the antagonistic muscles. In this case, a load is placed on the antagonistic muscles during the pedaling exercise.

Applied-voltage setting section 23B adjusts outputs of first electrodes 12 and second electrodes 13 so that the legs, which are predetermined parts of the body of the user doing the pedaling exercise, are electrically stimulated in relation to an exercise unit of the pedaling exercise. Applied-voltage setting section 23B adjusts strength of electrical stimulation related to an exercise unit, in accordance with the amount of a physical activity during the pedaling exercise. One example of the amount of a physical activity is the number of revolutions of crank 32. One example of an exercise unit is one revolution of crank 32. One example of strength of electrical stimulation is magnitude of an applied voltage. Thus, applied-voltage setting section 23B sets voltages applied to respective electrodes 12, 13, based on the number of revolutions of crank 32. An applied voltage is set such that its magnitude gradually increases toward the set voltage, in accordance with increase in the number of revolutions of crank 32. Note that the exercise unit may be a plurality of revolutions of crank 32 or a predetermined time of the pedaling exercise.

With reference t to FIGS. 1 and 2, the usage of pedaling exercise system 1 will be described.

A user sits on seat 40 and then starts doing the pedaling exercise with pedaling exercise machine 30. In this case, the user preferably does the pedaling exercise so that crank 32 rotates within a recommended rotation speed range of crank 32 (for example, a range between 30 rpm and 60 rpm per minute). When the number of revolutions of crank 32 is equal to or less than the predetermined number of revolutions RX, electrical stimulation apparatus 10 does not apply a voltage to respective electrodes 12, 13. After the number of revolutions of crank 32 exceeds the predetermined number of revolutions RX, electrical stimulation apparatus 10 applies predetermined patterns of voltages to respective electrodes 12, 13 at predetermined timings.

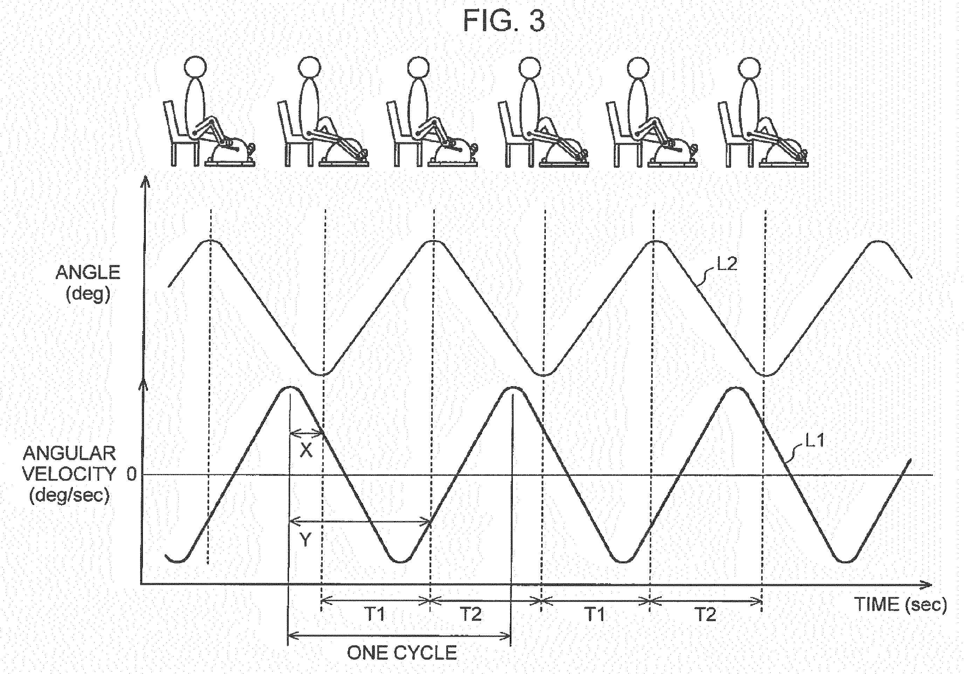

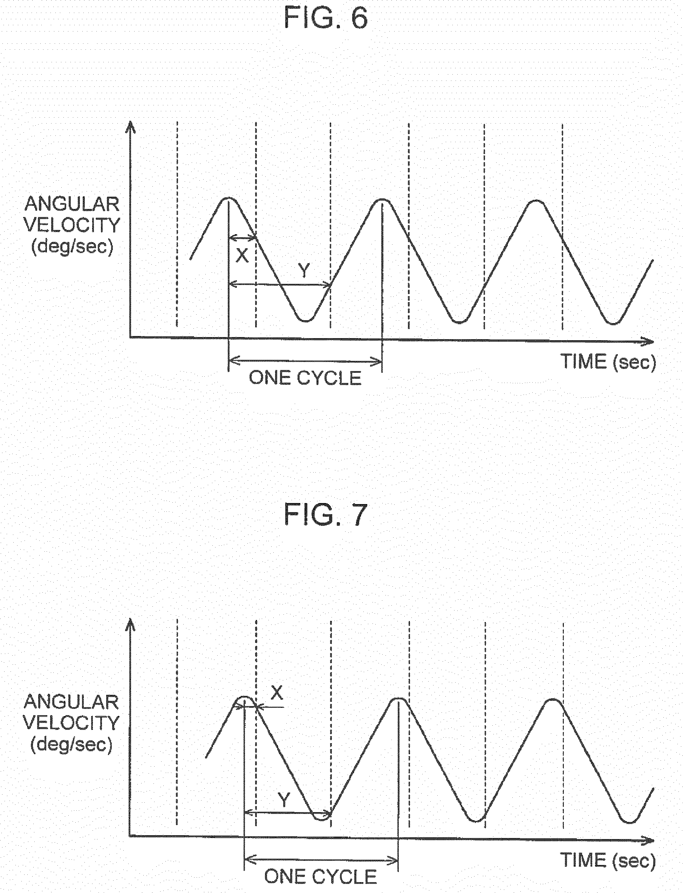

With reference to FIGS. 3 to 7, a method for setting timings of applying voltages to respective electrodes 12, 13 will be described. In FIGS. 6 and 7, lengths of one cycle are equal to each other between angular velocity AC acquired when crank 32 rotates at a high speed and angular velocity AC acquired when crank 32 rotates at a low speed, for the purpose of easy understanding in relation to a difference in a phase delay amount of angular velocities AC.

In a graph of FIG. 3, upper line L2 represents a change in an angle of a right knee joint of a user while the user is doing the pedaling exercise. In the graph of FIG. 3, lower line L1 represents a change in an angular velocity detected by angular velocity detector 14 in supporter 11. The angular velocity represented by line L1 corresponds to angular velocity AC acquired as a result of the filtering process.

As can be understood from lines L1, L2, a phase difference between the angle of the right knee joint and angular velocity AC. More specifically, the phase of angular velocity AC is delayed from the phase of the angle of the right knee joint. The main cause of this phase delay of angular velocity AC is the filtering process.

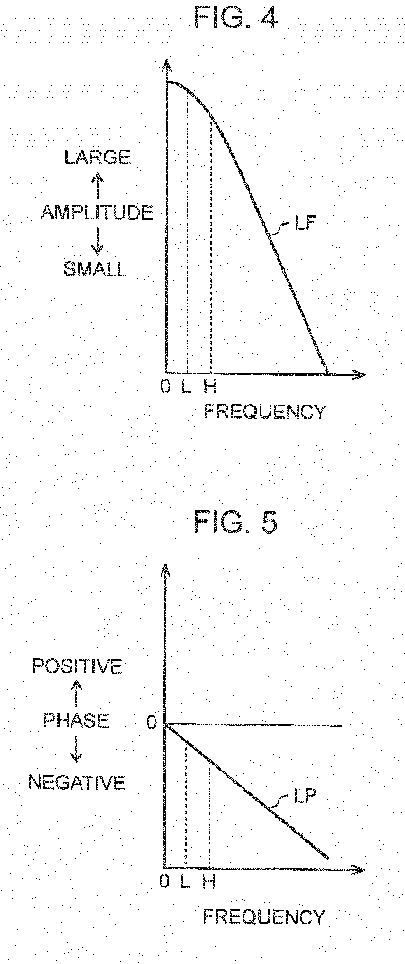

In some cases, for example, wobbling of the crank and wobbling of the pedals with respect to the crank arm may cause noise to be superimposed on angular velocities detected by angular velocity detectors 14. Further, when a distance between seat 40 and pedaling exercise machine 30 differs from a recommended distance, noise may be overlapped on angular velocities detected by angular velocity detectors 14. The recommended distance is set so that the user can do the pedaling exercise easily. For these reasons, when calculating angular velocity AC, electrical stimulation apparatus 10 removes noise from an angular velocity detected by each angular velocity detector 14 using the first and second filters to smooth the angular velocity. Solid line LF drawn on a graph in FIG. 4 and solid line LP drawn on a graph in FIG. 5 each show one example of a frequency response of the second filter.

When angular velocity AC is calculated by the filtering process, the frequency response (phase characteristic) of the filter influences more strongly a phase delay of angular velocity AC with respect to an angle of the right knee joint as the rotation speed of crank 32 becomes higher. In FIG. 4, for example, at frequency L, line LF indicates a decrease in the phase of angular velocity AC with respect to the angular velocity detected by each angular velocity detector 14 when crank 32 rotates at a low speed. In FIG. 5, at frequency L, line LP indicates a phase delay of angular velocity AC with respect to the angular velocity detected by each angular velocity detector 14 when crank 32 rotates at a low speed. In FIG. 4, likewise, at frequency H, line LF indicates a decrease in the phase of angular velocity AC with respect to the angular velocity detected by each angular velocity detector 14 when crank 32 rotates at a high speed. In FIG. 5, at frequency H, line LP indicates a phase delay of angular velocity AC with respect to the angular velocity detected by each angular velocity detector 14 when crank 32 rotates at a high speed. As illustrated in FIG. 5, the phase delay of the second filter increases with an increase in a frequency. This causes the phase delay of angular velocity AC to increase with an increase in a rotation speed of crank 32. As a result, as shown on graphs regarding temporal transition of angular velocity AC in FIGS. 6 and 7, the phase delay of angular velocity AC is smaller when crank 32 rotates at a low speed than when crank 32 rotates at a high speed. As can be understood from FIGS. 6 and 7, ratio X for a high-speed rotation of crank 32 is lower than ratio X for a low-speed rotation of crank 32, and ratio Y for a high-speed rotation of crank 32 is lower than ratio Y for a low-speed rotation of crank 32. Details of the ratios X and Y will be described later.

Therefore, voltage-application timing setting section 23A sets timings at which voltages are applied to respective electrodes 12, 13 in accordance with a degree of the phase delay of angular velocity AC caused by the filtering process and a degree of the phase delay of angular velocity AC due to the rotation speed of crank 32. More specifically, the relationship between an angular velocity of a lower limb and an angle of a knee joint is predetermined and is prestored in storage section 24. Voltage-application timing setting section 23A sets timings at which voltages are applied to respective electrodes 12, 13, based on the relationship between an angular velocity of a lower limb and an angle of a knee joint.

By simultaneously measuring an angular velocity of a lower limb and an angle of a knee joint, a phase difference between the angle of the knee joint and angular velocity AC can be acquired. Then, by simultaneously measuring angular velocities of lower limbs and angles of the knee joints at various rotation speeds of crank 32, a degree of the phase difference between angular velocity AC and the angle of the knee joint due to a rotation speed of crank 32 can be acquired. In the graph of FIG. 3, line L1 represents one example of a measurement result of an angle of a knee joint, and line L2 represents one example of a measurement result of an angular velocity of a lower limb. Line L2 has a peak (a maximum value) when the knee joint is bent maximally, and has a bottom (a minimum value) when the knee joint stretches maximally.

Storage section 24 stores the phase differences between lines L1 and L2 at the peak and bottom of line L2, as the relationships between an angle of a knee joint and an angular velocity of a lower limb. Further, the phase difference between lines L1 and L2 at the bottom of line L2 is stored as a first phase difference ratio (referred to below as a "ratio X") related to one cycle of angular velocity AC. The phase difference between lines L1 and L2 at the peak of line L2 is stored as a second phase difference ratio (referred to below as a "ratio Y") related to one cycle of angular velocity AC.

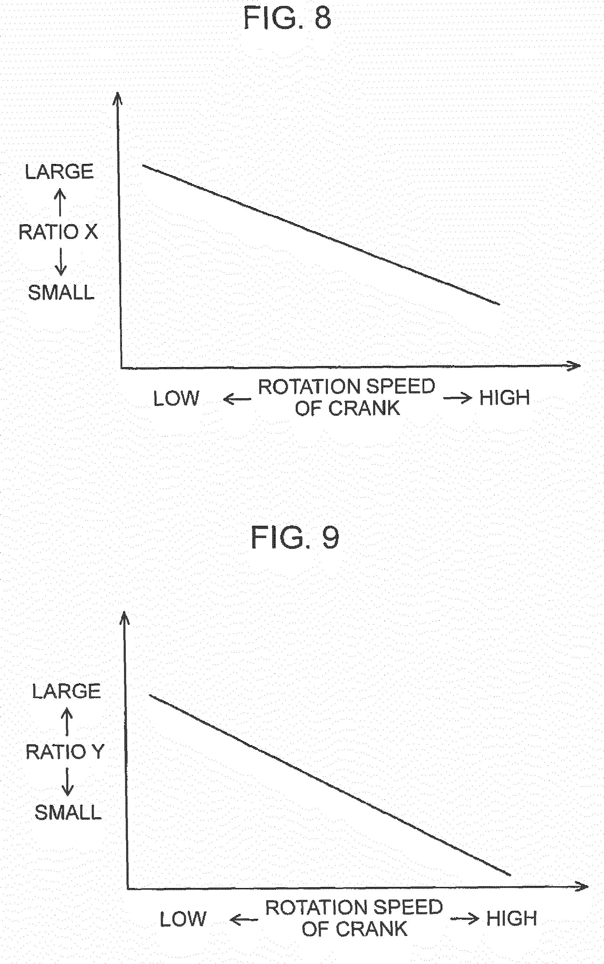

These ratios X, Y are stored in relation to various rotation speeds of crank 32. In one example, storage section 24 stores map MPX and map MPY. Ratio X is related to the rotation speed of crank 32 in map MPX as illustrated in FIG. 8. The ratio Y is related to the rotation speed of crank 32 in map MPY as illustrated in FIG. 9. In one example, in map MPX, ratio X decreases with an increase in the rotation speed of crank 32. In map MPY, ratio Y decreases with an increase in the rotation speed of crank 32.

Note that control section 21 may calculate ratios X, Y, based on a compensation equation, instead of based on map MPX of FIG. 8 and map MPY of FIG. 9. Ratio X can be defined as the compensation equation: X = A1 x R + B1, where R denotes a rotation speed, A1 denotes gradient of the straight line in map MPX of FIG. 8, and B1 denotes an intercept of the straight line. Ratio Y can be defined as the compensation equation: Y = A2 x R + B2, where A2 denotes gradient of the straight line in map MPY of FIG. 9, and B2 denotes intercept of the straight line.

Voltage-application timing setting section 23A sets a timing at which a voltage is applied to first electrodes 12, based on map MPX of FIG. 8 and a current rotation speed of crank 32. Likewise, voltage-application timing setting section 23A sets a timing at which a voltage is applied to second electrodes 13, based on map MPY of FIG. 9 and a current rotation speed of crank 32. As a result, when a lower limb stretches maximally during the pedaling exercise, voltage-application timing setting section 23A starts applying a voltage to the corresponding first electrodes 12 but stops applying a voltage to the corresponding second electrodes 13. When a lower limb is bent maximally during the pedaling exercise, voltage-application timing setting section 23A starts applying a voltage to the corresponding second electrodes 13 but stops applying a voltage to the corresponding first electrodes 12. In this way, electrical stimulation apparatus 10 can electrically stimulate quadriceps and biceps femorises at appropriate timings during the pedaling exercise. Furthermore, electrical stimulation apparatus 10 sets ratios X, Y in accordance with a rotation speed of crank 32. This can electrically stimulate quadriceps and biceps femorises at appropriate timings, in accordance with the pedaling exercise.

Further, over every first voltage-application period T1 (see FIG. 3), voltage-application timing setting section 23A applies a voltage to first electrodes 12 but does not apply a voltage to second electrodes 13. Over every second voltage-application period T2 (see FIG. 3), voltage-application timing setting section 23A applies a voltage to second electrodes 13 but does not apply a voltage to first electrodes 12. As illustrated in FIG. 3, first voltage-application period T1 corresponds to a time length between the application of the voltage to first electrodes 12 and the application of the voltage to second electrodes 13. Second voltage-application period T2 corresponds to time length between the application of the voltage to second electrodes 13 and the application of the voltage to first electrodes 12. Applying voltages in first application period T1 and second application period T2 in this manner enables a quadriceps femoris and biceps femoris to be electrically stimulated over the respective periods in which the quadriceps femoris and the biceps femoris stretch respectively.

A method for setting voltages applied to first and second electrodes 12, 13 will be described.

Storage section 24 stores information regarding the relationship between the number of revolutions of crank 32 and the voltages applied to respective electrodes 12, 13. When the number of revolutions of crank 32 is equal to or less than the predetermined number of revolutions RX, no voltages are applied to first and second electrodes 12, 13. According to this information, when the number of revolutions of crank 32 exceeds the predetermined number of revolutions RX, voltages are applied to first and second electrodes 12, 13. When the number of revolutions of crank 32 becomes RX + 1, an initial voltage lower than the set voltage is applied to first and second electrodes 12, 13. Then, as the number of revolutions of crank 32 increases, the applied voltage gradually increases toward the set voltage. In this way, a load on each leg gradually increases during the pedaling exercise, after the start of the rotation of pedals 35. This suppresses the quadriceps and biceps femorises from bearing heavy loads suddenly. Consequently, user discomfort due to an increase in the load of the pedaling exercise can be suppressed.

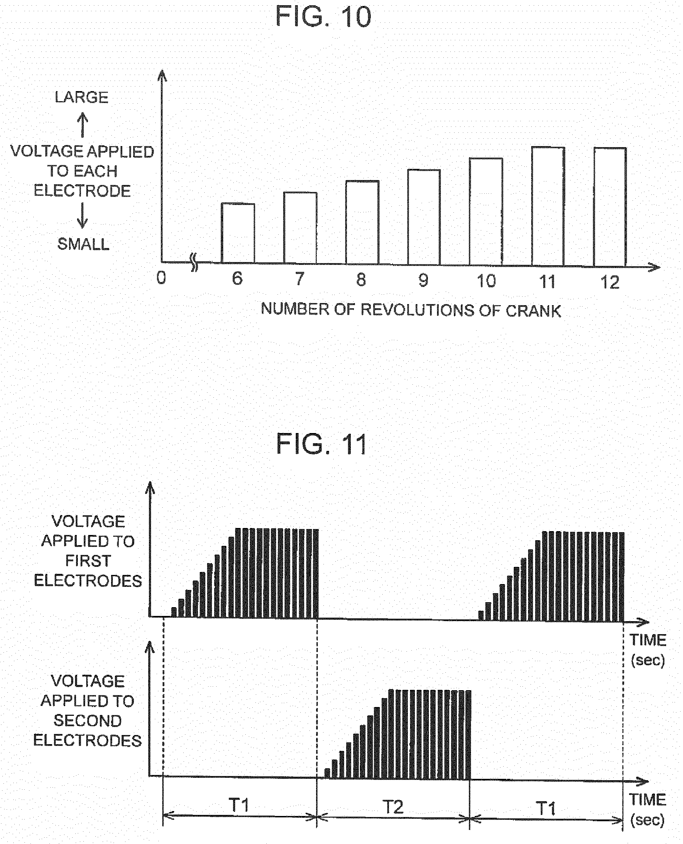

FIG. 10 illustrates one example of the above information. As can be understood from FIG. 10, when the number of revolutions of crank 32 is equal to or less than five, no voltages are applied to respective electrodes 12, 13. When the number of revolutions of crank 32 exceeds five, a voltage is applied to respective electrodes 12, 13. Further, when the number of revolutions of crank 32 is six, a voltage lower than the set voltage is applied to respective electrodes 12, 13. In the voltage application pattern in FIG. 10, the initial voltage applied when the number of revolutions of crank 32 is six is about 50% of the set voltage. When the number of revolutions of crank 32 falls within a range between seven and eleven, every time the number of revolutions of crank 32 increases by one, the voltage applied to respective electrodes 12, 13 increases by about 10%.

Storage section 24 prestores magnitude of the voltage applied to first electrodes 12 over first voltage-application period T1 and magnitude of the voltage applied to second electrodes 13 over second voltage-application period T2. FIG. 11 illustrates an example of these applied voltage values. As can be understood from FIG. 11, voltages applied to respective electrodes 12, 13 gradually increase during first half parts including start points of voltage-application periods T1, T2. Then, after the first half pats of voltage-application periods T1, T2 have passed, the voltages applied to respective electrodes 12, 13 are kept constant. Upper limit values of the voltages applied to electrodes 12, 13 over voltage-application periods T1, T2 are upper limit values of voltages illustrated in FIG. 10. Operation section 25 can change increasing rates of the voltages applied to respective electrodes 12, 13 according to an increase in the number of revolutions of crank 32, and increasing rates of the voltage applied to electrodes 12, 13 over voltage-application periods T1, T2.

Applied-voltage setting section 23B sets the voltages applied to respective electrodes 12, 13 at each number of revolutions of crank 32 using the voltage-application pattern in FIG. 10. In addition, applied-voltage setting section 23B sets voltages applied to electrodes 12, 13 over first voltage-application period T1 and second voltage-application period T2 using the voltage-application patterns in FIG. 11.

In this way, the voltages applied to respective electrodes 12, 13 are set low at the beginning of respective voltage-application periods T1, T2. Then, the voltages gradually increase with time. This further suppresses the quadriceps and biceps femorises from bearing heavy loads suddenly. Consequently, user discomfort due to an increase in the load can be further suppressed.

In this case, first voltage-application period T1 and second voltage-application period T2 are shorter when crank 32 rotates at a high speed than when crank 32 rotates at a low speed. However, the voltages applied to first electrodes 12 and second electrodes 13 have constant frequencies. Therefore, after the number of revolutions of crank 32 exceeds eleven, ratios in which the set voltage is applied to respective electrodes 12, 13 are lower when crank 32 rotates at a high speed than when crank 32 rotates at a low speed. In this case, the ratio in which the voltage applied to first electrodes 12 is expressed by the equation: (a time when the set voltage is applied to first electrodes 12 during every first voltage-application period T1)/(first voltage-application period T1) × 100. The ratio in which the voltage is applied to second electrodes 13 is expressed by the equation: (a time when the set voltage is applied to second electrodes 13 during second voltage-application period T2)/(second voltage-application period T2) x 100.

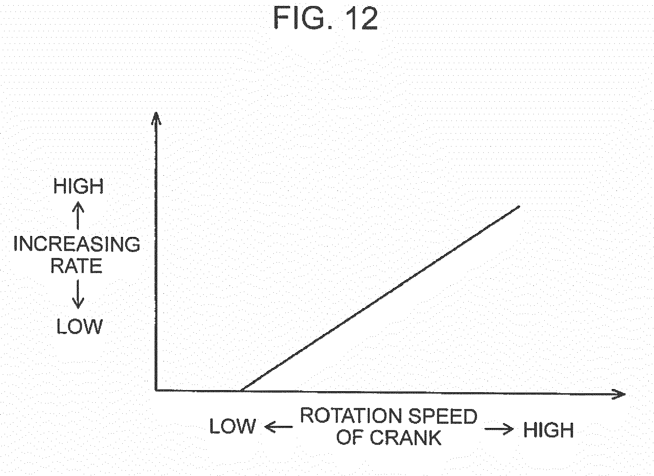

Therefore, applied-voltage setting section 23B adjusts increasing rates of the voltages applied to respective electrodes 12, 13 during voltage-application periods T1, T2 in accordance with the rotation speed of crank 32. More specifically, applied-voltage setting section 23B increases the increasing rates of the voltages applied to respective electrodes 12, 13 with an increase in the rotation speed of crank 32. Storage section 24 stores information regarding the relationship between the rotation speed of crank 32 and the increasing rates of the voltages applied to respective second electrodes 12, 13. This information may be stored in storage section 24 in a map format as illustrated in FIG. 12 or in a function format. In this exemplary embodiment, the increasing rate of the voltage applied to first electrodes 12 over first voltage-application period T1 is equal to the increasing rate of the voltage applied to second electrodes 13 over second voltage-application period T2. Thus, a single common map illustrated in FIG. 12 is used in the relationship between the rotation speed of crank 32 and the increasing rates of the voltages applied to respective electrodes 12, 13. Applied-voltage setting section 23B sets, using the map, for example as illustrated in FIG. 12, the increasing rates of the voltages applied to respective electrodes 12, 13 in accordance with the rotation speed of crank 32. This setting can reduce the difference in ratio between the set voltage applied to respective electrodes 12, 13 when crank 32 rotates at a high speed and when crank 32 rotates at a low speed.

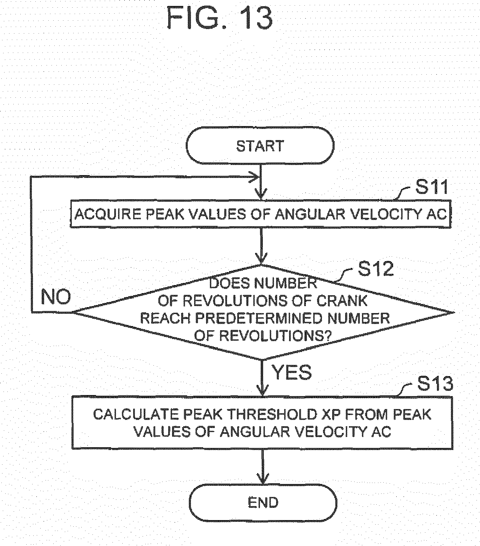

Various processes performed by control section 21 will be described below with reference to FIGS. 13 to 15.

Control section 21 performs a first process, a second process, and a third process. In the first process, control section 21 detects peaks of angular velocity AC within the period between a time when the pedaling exercise is started by a user and when the number of revolutions of crank 32 becomes the predetermined number of revolutions RX. In the second process that follows the first process, control section 21 sets timings at which voltages are applied to respective electrodes 12, 13. In the third process that follows the first process, control section 21 sets magnitude of voltages applied to respective electrodes 12, 13. The second process and the third process are simultaneously performed.

FIG. 13 illustrates steps of the first process. Control section 21 acquires a peak of angular velocity AC at step S11. The peak of angular velocity AC is stored in storage section 24. Control section 21 determines at step S12 whether the number of revolutions of crank 32 reaches the predetermined number of revolutions RX. When determining that the number of revolutions of crank 32 does not yet reach the predetermined number of revolutions RX, control section 21 returns this process to step S11. When determining that the number of revolutions of crank 32 reaches the predetermined number of revolutions RX, control section 21 calculates peak threshold XP from the plurality of peaks of angular velocity AC at step S13, and terminates the first process. In this process, after acquiring peaks of angular velocity AC at the predetermined number of revolutions of crank 32, control section 21 calculates peak threshold XP.

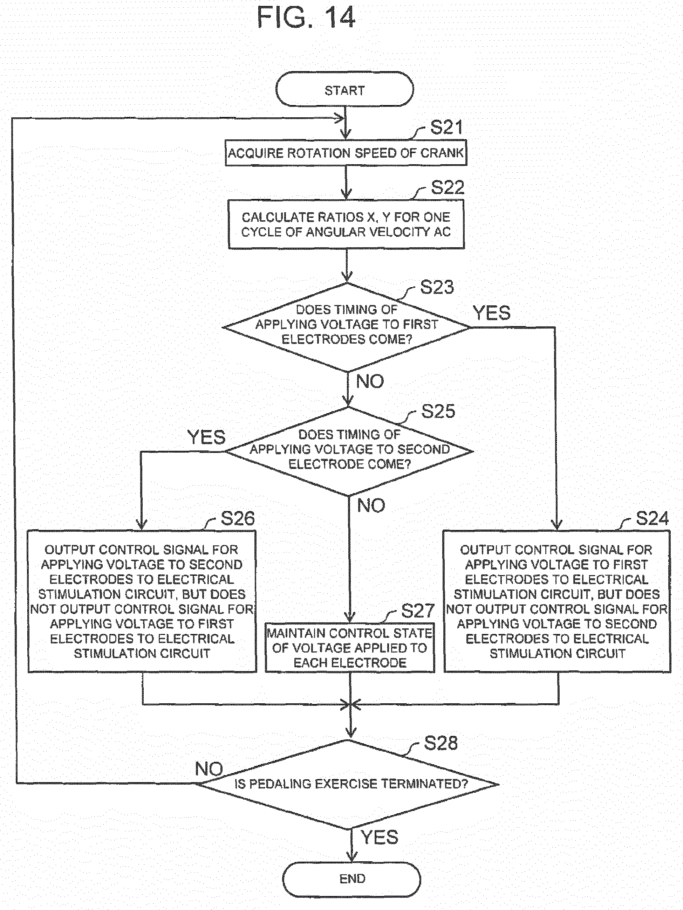

FIG. 14 illustrates steps of the second process. The second process for a right lower limb will be described with reference to FIG. 14. Note that the similar steps are applied to the second process for a left lower limb.

Control section 21 acquires a rotation speed of crank 32 at step S21. At step S22, control section 21 calculates ratios X, Y from the rotation speed of crank 32, using maps MPX, MPY. Accordingly, timings at which voltages are applied to respective electrodes 12, 13 are set. Control section 21 determines at step S23 whether the timing at which the voltage is applied to first electrodes 12 comes. When determining that the timing at which the voltage is applied to first electrodes 12 comes, the control section 21 outputs a control signal for applying the voltage to first electrodes 12 to electrical stimulation circuit 27 at step S24. However, the control section 21 does not output a control signal for applying the voltage to second electrodes 13 to electrical stimulation circuit 27. As a result, first electrodes 12 start electrically stimulating the quadriceps femoris. When the number of revolutions of crank 32 is RX + 1, second electrodes 13 do not start electrically stimulating the biceps femoris. When the number of revolutions of crank 32 exceeds RX + 2, namely, after the voltage is started being applied to second electrodes 13, second electrodes 13 stops electrically stimulating the biceps femoris.

When determining that the timing at which a voltage is applied to first electrodes 12 does not yet come, control section 21 determines at step S25 whether the timing at which the voltage is applied to second electrodes 13 comes. When determining that the timing at which the voltage is applied to second electrodes 13 comes, at step S26 control section 21 outputs the control signal for applying the voltage to second electrodes 13 to electrical stimulation circuit 27. However, the control section 21 does not output the control signal for applying the voltage to first electrodes 12 to electrical stimulation circuit 27. As a result, second electrodes 13 starts electrically stimulating the biceps femoris, whereas first electrodes 12 stop electrically stimulating the quadriceps femoris.

When determining at step S25 that the timing at which the voltage is applied to second electrodes 13 does not yet come, at step S27 control section 21 maintains a control state of the voltages to first and second electrodes 12, 13.

Control section 21 determines at step S28 whether the pedaling exercise is terminated. Control section 21 makes the determination at step S28, based on the period of the pedaling exercise which has been set by operation section 25. When determining that the pedaling exercise is not terminated, the control section 21 returns this process return to step S21. When determining that the pedaling exercise is terminated, the control section 21 terminates the second process.

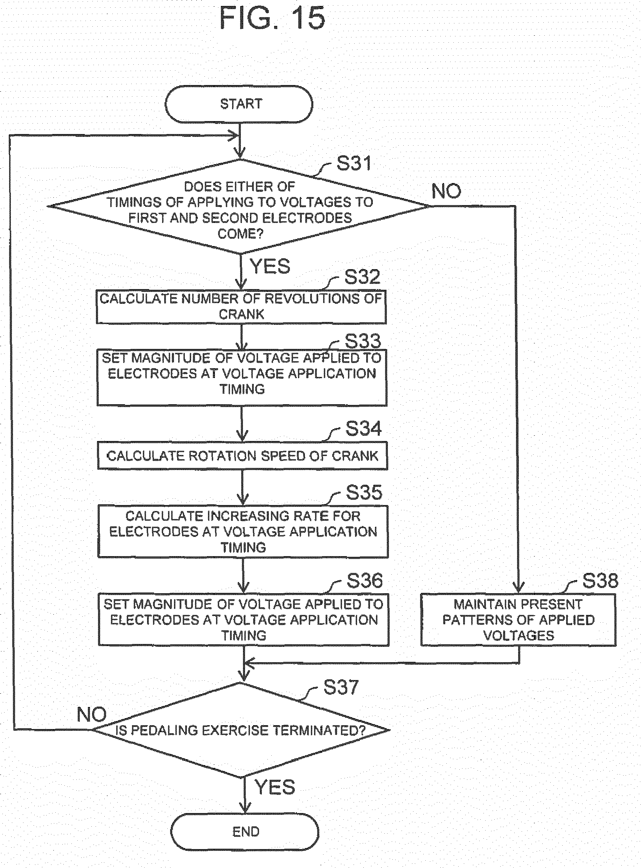

FIG. 15 illustrates steps of the third process. Control section 21 determines at step S31 whether either of the timings at which the voltages are applied to electrodes 12, 13 comes. When determining that either of the timings at which the voltages are applied to respective electrodes 12, 13 comes, at step S32 control section 21 calculates the number of revolutions of crank 32 at this time. At step S33, control section 21 sets magnitude of a voltage applied to first electrodes 12 or second electrodes 13, whose voltage application timing comes, based on the number of revolutions of crank 32.

At step S34, control section 21 calculates a rotation speed of crank 32. At step S35, control section 21 calculates an increasing rate of the voltage applied to ones of first electrodes 12 or second electrodes 13, whose voltage application timing comes, based on the rotation speed of crank 32. At step S36, control section 21 sets magnitude of the voltage applied to first electrodes 12 or second electrodes 13, whose voltage application timing comes, based on the increasing rate calculated at step S35.

Control section 21 determines at step S37 whether the pedaling exercise is terminated. When determining that the pedaling exercise is not terminated, control section 21 returns this process to S31. When determining that the pedaling exercise is terminated, control section 21 terminates the third process.

When determining at step S31 that neither of the timings at which the voltage is applied to first electrodes 12 and at which the voltage is applied to second electrodes 13 comes, control section 21 maintains the present patterns of the voltages applied to respective electrodes 12, 13 at step S38 so as to proceed to the determination at step S37.

### MODIFICATION

The foregoing exemplary embodiment has provided examples of aspects of a control device for an electrical stimulation apparatus, an electrical stimulation apparatus, and a pedaling exercise system in the present disclosure, and therefore is not intended to limit the present disclosure. A control device for an electrical stimulation apparatus, an electrical stimulation apparatus, and a pedaling exercise system in the present disclosure may employ aspects of some modifications of the foregoing exemplary embodiment which will be described below or combinations of two or more of these modifications which are not mutually contradictory.
- In the foregoing exemplary embodiment, control section 21 may calculate the number of revolutions and rotation speed of crank 32, based on a detection signal from a crank sensor. Moreover, control section 21 may set voltages applied to respective electrodes 12, 13, based on the detection signal from the crank sensor.
- In the foregoing exemplary embodiment, control section 21 may be provided inside pedaling exercise machine 30. In this case, pedaling exercise machine 30 is electrically connected to first electrodes 12, second electrodes 13, and angular velocity detectors 14 in supporters 11. Furthermore, controller 20 and pedaling exercise machine 30 may be integrated with each other.
- In the foregoing exemplary embodiment, only one angular velocity detector 14 may be provided in either of supporters 11.
- In the foregoing exemplary embodiment, state detector 22 may include a bottom detector that detects a bottom of a detection signal from each angular velocity detector 14. Control section 21 may calculate an amplitude of angular velocity AC from the peak and bottom of angular velocity AC.
- In the foregoing modification, voltage-application timing setting section 23A may set the timings at which the voltages are applied to respective electrodes 12, 13, based on ratios X, Y preset for the amplitude of angular velocity AC.
- In the foregoing modification, when the bottom detector is provided to state detector 22, peak detector 22C do not necessarily have to be provided to state detector 22. In this case, control section 21 calculates one cycle of angular velocity AC, and the number of revolutions and rotation speed of crank 32, based on the bottom detected by the bottom detector.
- In the foregoing exemplary embodiment, control section 21 may calculate one cycle of angular velocity AC, based on zero crossing points of angular velocity AC, instead of based on neighboring peaks of angular velocity AC.
- In the foregoing exemplary embodiment, filter switch 22F may further include a third filter whose frequency response differs from frequency response of the first filter and the second filter. In this case, filter switch 22F may switch between filters, at least once, to smooth an angular velocity detected by angular velocity detector 14 over the period between the start and the termination of the pedaling exercise done by a user.
- In the foregoing exemplary embodiment, control section 21 may compensate for the increasing rates of the voltages applied to respective electrodes 12, 13 which are related to an increase in the number of revolutions of crank 32, based on a rotation speed of crank 32. Applied-voltage setting section 23B makes this compensation so that the increasing rates of the voltages applied to respective electrodes 12, 13 increase with an increase in the rotation speed of crank 32. With this compensation, when crank 32 rotates at a high speed, voltages applied to respective electrodes 12, 13 at a small number of revolutions of crank 32 are set as their set voltages.
- In the foregoing exemplary embodiment, applied-voltage setting section 23B may set the maximum values of voltages applied to respective electrodes 12, 13 in a variable manner, in accordance with the number of revolutions of crank 32. For example, after the set voltages have been applied to respective electrodes 12, 13, applied-voltage setting section 23B may gradually decrease the maximum values of the voltages applied to respective electrodes 12, 13 from the set voltages according as the number of revolutions of crank 32 increases. Alternatively, applied-voltage setting section 23B may vary the maximum values of the voltages applied to respective electrodes 12, 13, in accordance with the number of revolutions of the crank 32.
- In the foregoing exemplary embodiment, a user may do the pedaling exercise with both arms. In this case, supporters 11 are attached to the right and left upper arms respectively. In this case, first electrodes 12 is disposed at a location related to the biceps brachii muscles, for example, whereas second electrodes 13 is disposed at a location related to the triceps brachii muscles, for example. In addition, angular velocity detector 14 is disposed at a location related to the biceps brachii muscles, for example.

The electrical stimulation apparatus and the pedaling exercise system of the present disclosure may be applied to any electrical stimulation apparatus and any pedaling exercise system for household use, professional use, or any other uses.

## Claims

1. A control device for an electrical stimulation apparatus, comprising:
a control section that adjusts an output of an electrode, the electrode giving electrical stimulation to a predetermined part of a body doing a pedaling exercise, the predetermined part being at least one of a leg and an arm,
wherein the control section adjusts the output of the electrode so that the electrical stimulation is given to the predetermined part in relation to an exercise unit of the pedaling exercise, and adjusts strength of the electrical stimulation related to the exercise unit, in accordance with an amount of a physical activity of the body during the pedaling exercise.

2. The control device for an electrical stimulation apparatus according to claim 1, wherein the control section adjusts the strength of the electrical stimulation related to the exercise unit so that the strength of the electrical stimulation increases according as the amount of the physical activity of the body increases during the pedaling exercise.

3. The control device for an electrical stimulation apparatus according to claim 2, wherein when the strength of the electrical stimulation is set by an operation section, the control section adjusts the strength of the electrical stimulation related to the exercise unit so that an initial value of the electrical stimulation related to the exercise unit becomes smaller than the set strength and that the strength of the electrical stimulation gradually increases toward the set strength according as the amount of the physical activity of the body increases.

4. The control device for an electrical stimulation apparatus according to claim 3, wherein the control section adjusts an increasing rate of the strength of the electrical stimulation when the strength of the electrical stimulation is gradually increased toward the set strength, in accordance with a rotation speed of a crank in a pedaling exercise machine.

5. The control device for an electrical stimulation apparatus according to one of claims 1 to 4, wherein the control section causes the electrode to output the electrical stimulation related to the exercise unit by applying a pulse voltage to the electrode.

6. The control device for an electrical stimulation apparatus according to claim 5, wherein the control section gradually increases the pulse voltage during a first half of a period of the exercise unit, the first half of the period containing a starting point of the exercise unit.

7. The control device for an electrical stimulation apparatus according to claim 6, wherein the control section adjusts the increasing rate of the voltage when the pulse voltage is gradually increased, in accordance with a rotation speed of the crank of the pedaling exercise machine.

8. An electrical stimulation apparatus comprising:
the control device for an electrical stimulation apparatus according to one of claims 1 to 7; and
the electrode.

9. A pedaling exercise system comprising:
the electrical stimulation apparatus according to claim 8; and
the pedaling exercise machine.
